# EUROPEAN PATENT APPLICATION

(11) **EP 1 887 078 A1**
(43) Date of publication of application: **13.02.2008**
(21) Application number: 07106110.5
(22) Date of filing: 13.04.2007
(51) Int. Cl.: C12N 1/20, C12N 1/22, C12N 1/38, C12N 11/12, C02F 3/04, C02F 3/10, C02F 3/32

(54) **Bacteria inoculation system with earthworm humus, used as a treatment of contaminated water (inoculom)**

(30) Priority: 11.08.2006 NZ 54911906
(71) Applicant: Villagra Fuentes, Alex Manuel, 1616 Cerillos, Santiago (CL)
(72) Inventor: Villagra Fuentes, Alex Manuel, 1616 Cerillos, Santiago (CL)
(74) Representative: Lahidalga de Careaga, Jose Luis

(57) **Abstract**

The invention concerns a system to form different bacterial floras that serves as a decontamination treatment of residual waters. The system uses earthworms humus of any specie of earthworm, as a detonator of the different bacterial flora that may form depending of the type of residual water that is pretended to decontaminate, which is located in a celluloid support material in the first layer of the system as a support means for the stuck bacterial growth, inert material on the second layer of the system (intermediate) that can be stones or plastic, that may permit the formation of a bacterial flora the supports the degradation of the organic material and finally, on the inferior part (third layer) other inert material that allows to form an air chamber over the system floor, for which it is connected to the surface by vertical tubes through the different layers and horizontal tubes that are connected to the exterior through its walls of the system, this last layer can be made with prefabricated concrete material, stones of different sizes or plastic, that allow an aerobic material and a fast drainage of the water to the exterior of the system. The invention further concerns an apparatus for treating waste water or other waste liquids, including semi-liquids.

## Description

This system (INOCULOM) consists in using the humus of earthworms humus to inoculate bacteria, usually different types of bacteria, using several types of mediums or fixation materials. The particularity of this system is that the bacteria are to be inoculated using different liquid or semi liquid residues, which when in contact with the humus and with the medium material, begins to generate a bacterial flora specialized in degrading the type of liquid or semi liquid residue that is being used.

### DESCRIPTION

In one aspect, the present invention consists in an apparatus for treating waste water or other waste liquids (including semi-liquids), the apparatus comprising:
a tank or other container, said container containing:
   a first layer comprising humus of earthworms;
   a second layer comprising a cellulose or celluloid material, for example, sawdust, underlying the first layer: and
   a third layer comprising a substantially inert material defining a porous bed
   underlying the second layer;
means to distribute the waste liquid onto the first layer for the waste liquid to perculate through the layers to the bottom of the container; and
means to drain or draw treated liquid from the container.

Preferably, the apparatus includes means to aerate the container, particularly a selected part or parts of the container, more particularly the bottom region of the container.

In another aspect, the present invention consists in a system to form different bacterial floras that serve as a decontamination treatment of residual waters, that is **characterised** because it uses **EARTHWORM'S HUMUS** of any specie of earthworm, as a detonator of the different bacterial flora that may form depending of the type of residual water that is pretended to decontaminate, which is located in the first layer of the system, being this last one over a celluloid material which is the second layer that works as a medium for the bacterial growth and a formation of a bacterial flora. On the third layer of the system there's inert material that can be stones or plastic that allow as well the formation of a bacterial flora that helps the degradation of organic material; this three last layers are found inside the tank; the tank's base is made up of inert material that allows taking an air chamber over the system's floor (false bottom) which is connected to the surface by vertical tubes through the different layers and horizontal tubes that are connected to the exterior through the walls of the system; this last layer can be made with prefabricated concrete material, stones of different sizes or plastic, that may allow an aerobic material and a fast drainage of the water to the exterior of the system.

In any aspect of the invention, the humus preferably includes earthworms. The activity of the earthworms adds to the existing humus.

In another aspect, the present invention consists in a method of treating liquid wastes using the apparatus or system as defined above or using the steps provided by such apparatus or system

This bacteria inoculation process can be used for different ends, but the most important, is to clean water contaminated with organic material, from which can be mentioned sewer water (domestic), industrial water, and specially all water that presents organic contamination. For the industrial water, it can be mentioned the food industry discharges, slaughterhouses, dairies, vineyards and agricultural industries, etc...

The main advantage of this bacterial flora inoculation system is that it is triggered the same way (using earthworm humus) for different liquid or semi liquid wastes.
Two examples can be mentioned as example of the above:
a.- Slaughterhouse wastes: The slaughterhouses' liquid wastes are made up of mainly organic material, especially blood, green waters, excrement and fats, which are the main organic contaminants that present such industrial establishments. Therefore, to degrade in an organic form these types of wastes, is necessary to form an active bacterial flora, specialized in degrading of (feeding on) the organic material living in the residual discharge of the slaughterhouse. The inoculation system is composed of a recipient that the sawdust and earthworm humus is located in its main part contaminated water is poured in its superior part and it is passed through this material (sawdust and humus) and in this contact a bacterial flora specialized in this type of liquid waste begins to form.
b.- Dairy wastes: The liquid wastes of a dairy company are composed mainly of lactose, milk proteins, flour, vegetable fat, oil and biodegradable detergent, which are the main organic contaminants of this type of industry. In order to degrade the organic material living in the water, it must be in contact with earthworm humus in its sawdust medium, with the contaminated water that is disposed by the company.

The aforesaid two cases will form different types of bacterial flora in the sawdust medium, yet the two cases started in the same way, as it is said, by putting in contact the contaminated water with the earthworm humus.

This bacteria inoculation process needs to be separated from the majority of solids suspended that come from the residual water, this with the end of not perming the bed of the medium (sawdust) and in this way, damage the formation of the aerobic bacterial flora. If there are many solids accumulated in the sawdust medium pools of contaminated water will be formed, which may provoke odors and an anaerobic (absence of oxygen) instead of aerobic (presence of oxygen) bacterial flora will tend to form in the bed of the sawdust. The anaerobic bacterial flora provokes odor problems in the water that filters through the medium, which is not recommendable to happen, because the water that is pretended to filter will come out of the system with odors.

The medium that is to be used to inoculate different bacterial flora has to be in base of a celluloid material, this can be found in different percentage in the medium (inoculation or fixation vehicle), this is to say, alfalfa can be exist in the medium in a 60% and the celluloid material in a 40% or it can exist in other concentrations, but the most important is that there exists a celluloid material percentage that serves as a shelter to the earthworm humus.

It is very important to make a separation of the bigger quantity of solids that may come from contaminated water, this not to overwhelm the medium of the bacterial flora, yet that this may provoke a formation of anaerobic bacteria, making serious odor problems.

This bacterial flora inoculation method based on earthworm humus, has as the main use the treatment of industrial liquid and solid wastes.

### Use of INOCULUM in contaminated water treatment.

This system (INOCULOM) is used to inoculate different bacterial flora, depending on the liquid waste that is going to be used, as it is said: is a liquid waste is to be used from a dairy company, the flora that will activate this method (INOCULOM) is different from the one that will activate to take care of the wastes of a vineyard or slaughterhouse.

We are going to call the water that is used to inoculate the bacterial flora **Primary Water (9)** and when the flora is already activated in the water that is going to be decontaminated will be called **Effluent (9)** and the one that already passed through the INOCULOM system will be called **Affluent**.

This method (INOCULOM) is preferably composed of the following parts, reference being made to the accompanying drawings.

This method (INOCULOM) needs to be confined in a pond or tank or other container that has to be perforated in the inferior part in order to evacuate the **Primary Water (9)** that is being used to inoculate the bacterial flora. This water is scattered in the superior part on an homogeneous form, with the intention to cover all the system and it is collected by the inferior part. This Water could be used several times, but it is going to depend on the amounts of disposal and the conditions of the water flowing in the inferior part.

The Primary Water (9) that is distributed in the superior part, passes on a gravitational form in all the layers of the system (INOCULOM) and it is recollected in the inferior part by channels with slopes (7). This pond is made of walls that can be handcrafted, concrete made, fiberglass, wood, steel, etc, there does not exist exactly a defined material to build a Bacteria Inoculator (INOCULOM), it is only necessary that there exist walls capable of maintaining the medium material that makes its interior. The floor (eg, concrete base (4)) of the pond has also no defined material, whatever material that resists the requirements may serve, for example compression of the material that makes the Bacteria Inoculator (INOCULOM) and impermeability.

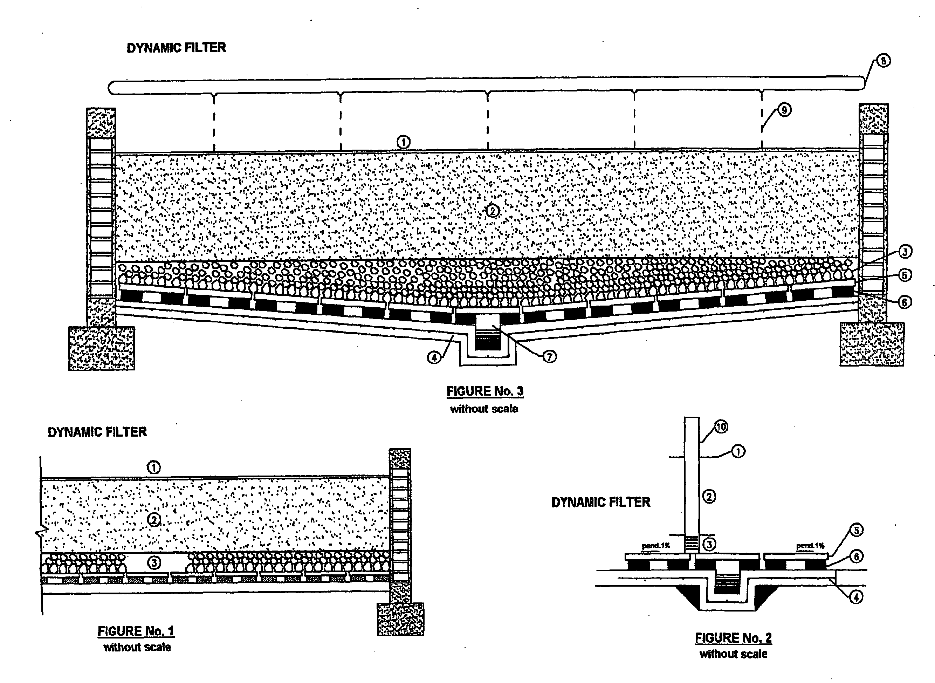

The Bacteria inoculator (INOCULOM) is made of earthworm humus (any type of earthworm) in its first layer (1), mixed with a celluloid material that may be sawdust, wood shaving, etc (as shown in Fig. No.1). This first layer also serves as a filter in case that the Bacteria inoculator (INOCULOM) is used as a water treatment.

The earthworm humus (1) (any type of earthworm) is the trigger to form different bacterial flora, as it is said, the main feature of the Bacteria Inoculator system (INOCULOM) is to put in contact the earthworm humus with the liquid waste that is pretended to dissolve, this liquid waste can be: sewer wastes, liquid industrial waste, etc...

The formation of the bacterial flora is made in several layers of the Bacteria Inoculator system (INOCULOM), there being the main one the first layer of sawdust and earthworm humus that is activated, and which is aerobic. On the other layer of inert material (rocks, plastic, etc.) whose main objective is to evacuate rapidly the water from the system, a Bacterial Flora of aerobic character needs to be formed.

The bacteria inoculation medium is the second layer of the system, which is made up of celluloid material (2) that can be sawdust or wood shaving. The thickness of this celluloid material is approximately 70 cms. deep, in which micro organisms produced by the earthworms' humus (1) and annelids (earthworms) inhabit together. Under this second layer there is a third layer of inert material, as for example: quarry stones and rocks (3). On these further layers an anaerobic bacterial layer is also formed that serves to keep up degrading the organic material that the contaminated water (effluent) presents.

On the main medium of bacteria inoculation (2) (humus and sawdust) an ample bacterial flora is generated, that in case of using it to decontaminate water, may obtain a deep contamination removal. The following reductions can be obtained in sewer water: 95% of the BDO's, 93% of the volatile suspended solids, 96% of the volatile solids, 70% of nitrogen and 70% of phosphorus.

Earthworms can be added in the earthworms humus (1), adults or juvenile (larvae), which are increase the formation of humus because they are continuously defecating as they feed on the organic material in the contaminated water, which is retained in the first layers of the Bacterial Inoculation system. Therefore, in the earthworm humus (1), adult earthworms as well as larvae can be found in different species.

The sawdust (2) functions as a medium for the bacterial flora and at the same time as a food supplement in the eventual case that the contamination charge of the water, may not be sufficient to feed the as it is normal. The sawdust (2) serves as food to the earthworms due that in time it gets degraded by the action if the micro organisms. The earthworms normally feed their weight in one day.

The third layer of the medium, is, for example, made up of quarry stones and rocks (3), the quarry stones are placed in the inferior part and the lesser ones are placed in the superior part. A bacterial flora is formed on the rocks that digests the organic material present in water that passes through it and that was not retained in the superior layers of the Bacterial Inoculator,

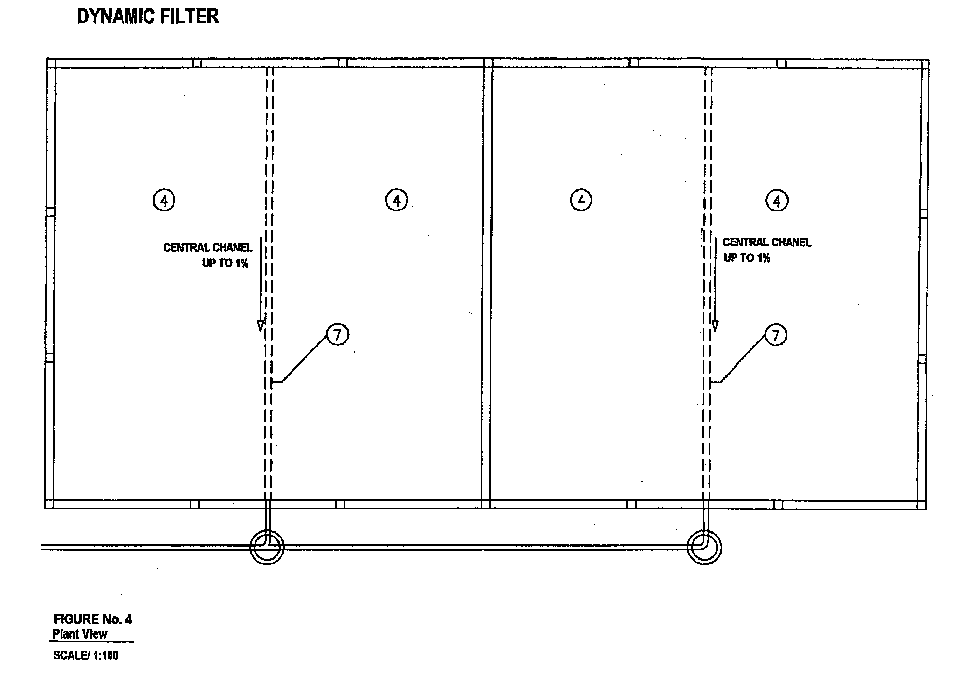

The floor of the Bacterial Inoculator (INOCULOM) (as shown in Figs. N°2 and N°4), also denominated as false bottom, consists in a concrete floor (4) with certain inclination for the water to flow and on its top there exist bricks (5) aside of the supports (6) that can be any resistant or inert materials (stainless steel bars, bricks, paving stones, etc.). These bricks (5) can be in different sizes, separated 2, 3, or 4 cms. between each other. The stones (3) are placed on top bigger than the support, principally the ones whose diameter is bigger than 4 cms., the different layers of the system and II) to create a sole water guide, in a form of a box that constitutes the treated water emitter (7), that is conducted to the exterior of the system (INOCULOM).

The double bottom is connected to a vent (10) that has as a main function to oxygenate the inferior part of the Bacterial Inoculator (INOCULOM), which vent consists in a perforated tube in its inferior part and the upper part is opened. This tube is located over the false bottom, on the intersections of four bricks (5) and the perforations that are made in the inferior part which are not bigger than 1 inch of diameter. This vent permits an exchange of oxygen in the exterior part of the bottom of the Bacterial Inoculator (INOCULOM).

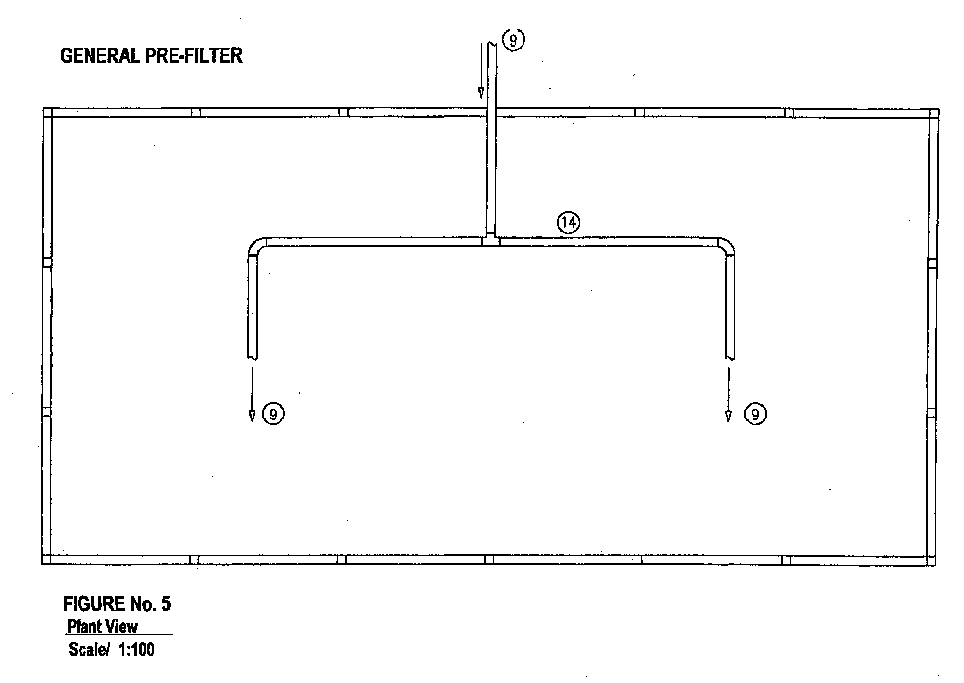

The contaminated water distribution over the Bacterial Inoculator (INOCULOM) can be made in different ways, as for example, using a PVC perforated pipe network (8) (as shown in Fig. N° 3) sprinklers, stems, nozzles, open channels, etc. the means used for the distribution of the contaminated water (9) are not very important, but that the distribution is preferably uniform over the filtering bed, therefore, every method works if it fulfills this condition.

For the construction of the thick building of the Bacterial Inoculator (INOCULOM), there is no unique material that can be used, any material (concrete, interknit, brick, etc.) existing in the market that has the impermeability and capacity characteristics to support the weight of the different layers of the INOCULOM can be used.

The Bacterial Inoculator (INOCULOM) used as a biological system in the contaminated water treatment, degrades all of the organic solids from the contaminated water, without producing unstable mud as the rest of the traditional treatments.

The affluent of the INOCULOM used as water treatment has a very low absorption (is transparent) which permits the elimination of pathogen microorganisms, using later any disinfection system (ultraviolet radiation, chlorine, ozone, etc.).

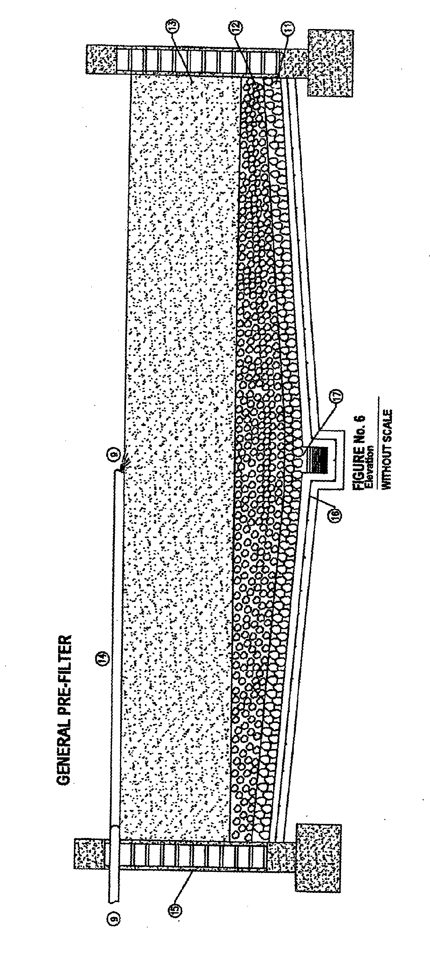

Before the contaminated water enters the INOCULOM used as a water treatment, the water has to be or should be filtered, especially the Liquid Industrial Residues (Riles), due specially to the excess of solids suspended that may overwhelm the bed of the system, causing flooding, which can possibly cause the earthworms or larvae in the INOCULOM to drown, not permitting to add more earthworm humus. To filter the contaminated water (crude water) different filtering systems can be used, as for example: rotating filters, parabolic filters, decantators, etc. And to filter in a finer form, a sawdust based system can be used, similar to the Baterial Inoculation (INOCULOM) system, but without humus, or earthworms, which permits the passage of more water per square meter. This filtering system will be named General Pre-filter (P.G.) Figures N°5 and N°6), composed of the following filtering layers: in the inferior part of the layer of the quarry stones or rocks (11) from 2" to 4" which has a thickness of 40 cms, then gravel is added (12) of 0,5" which has a thickness of 25 cms, and shavings are added after this layer (13), with a thickness of 55 cms from the gravel layer. The contaminated water (9) over the General Pre-filter (P.G.) can be distributed (14) with perforated pipes, stems, nozzles, sprinklers or just discharged over a part of the surface of the General Pre-filter (P.G.). In the event that the shavings get filled, in the discharge place, only the water starts to expand in places not yet filled therefore the water discharge over this General Pre-filter (P.G.) does not posses in an exclusive way, the important thing is to discharge it in a gentle form over the General Pre-filter surface, so it does not make any holes. This General Pre-filter (P.G.) is confined inside a pond or tank or other container that has the same physical characteristics that the Bacterial Inoculation (INOCULOM), as it is said, walls can be built (15) from the General Pre-filter (P.G.) in craftsmanship, on any of its types (bricks, blocks, etc.) and the concrete base (16) in reinforced concrete with slopes (17), that permits to evacuate the contaminated water that was disposed in the upper part through the inferior part of the General Pre-filter (P.G.).

### State of art in Contaminated water Treatment.

The served water and Industrial residue organic liquid (RILES) treatment services are divided into two categories, anaerobic (absence of oxygen) and aerobic (presence of oxygen). For each of these categories, there exists a wide variety of treatments. For example inside of the anaerobic, the most common are the anaerobic lagoon and the closed digester; and inside the aerobic, the most used are the denominated: activated mud and the pre-strained filters.

This new treatment based in the Bacteria Inoculator (INOCULOM) is an aerobic system, since it needs oxygen to function correctly and because it constitutes in a certain way a filtering medium, it can be classified as: Biological System for the Contaminated Water treatment in base on a Pre-straining filter modification.

### Pre-straining Filters

The traditional pre-straining filters are composed of stone supports, over which a certain flow of contaminated water lands through a distribution system. A Bacterial flora with different types of microorganisms is formed in the stones coming from the contaminated water that direct the organic material. Yet the pre-straining traditional filters do not give the high index of organic material removal that the mud activated process gives; nevertheless they have its advantages regarding space, energy and the capacity to resist disturbances.

The pre-straining filters have two big disadvantages, which are:

### a) They produce a great amount of waste mud.

The waste mud, as usual, reach from 0,22 to 0,66 lb of solids per Ib of DBO₅ removed in a system of domestic water wastes. About industrial wastes, the production of mud can be as high as 0,4-0,5 Ib/Ib of DBO₅ removed if the content of carbohydrate waste is high, or as low as 0,05-0,1 Ib/Ib of DBO₅ removed if the organic materials are volatile acids or alcohols.

### b) The filtering bed gets impermeable.

The rocks as well as the revolving disks, get impermeable with the fat that the contaminated water contains. Due to this, the filter efficiency is reduced considerably.

### Treatment with active muds (aerobic system).

This treatment system is actually the most used, because it is smaller, and it operates with more elevated organic charges and in varied weather conditions. Commonly, the air is supplied through diffusers placed underneath the surfaces. In some plants, pure oxygen is supplied instead of air, which may reduce the volume of the incubator even more.

The most important disadvantages of this type of treatment are the following:

### a) It produces a great amount of waste mud.

The mud production is significantly greater than for other treatment systems, which entails a more complex operation of the plant. A portion of the waste muds are recycled over the aeration ponds and the other part has to be stabilized with some type of treatment.

### b) Elevated operational costs.

This system needs that the aerators are constantly working in the ponds of aeration, supplying pure oxygen or air to the contaminated water, which is traduced in a high consumption of energy.

Due to the complex muds methods, pumps are needed to suck muds from the bottom of the aeration ponds and transport them to places where they are going to be stabilized - which makes considerably more expensive the operation costs of the plant.

### New Treatment based on the Bacterial Inoculator (INOCULOM)

This new served water and liquid organic Industrial wastes treatment system has the following **ADVANTAGES:**

### a) It does not produce unstable muds.

In the served water treatment and liquid organic industrial wastes traditional processes, they have as solid subproducts (muds) that are produced by the contaminant elimination of crude water, for which it has to be made a secondary disposal.

This new served water and liquid industrial residues treatment system, degrades the totality of the organic solids coming from the contaminated water, without producing unstable muds unlike the rest of the traditional systems, because the earthworms in the medium of the Bacterial Inoculation (2) (humus and sawdust) degrades the totality of the solids that are retained in this means, transforming it in a stable odorless material, like **humus.**

The sewer water treatment **based on the Bacterial Inoculator (INOCULUM)** does not need before any type of solid organic decantator, it is only necessary, to install a bar chamber to retain the inorganic solids coming from the sewage water which may cover the pipes or the distribution system.

### b) The filtering riverbed does not get impermeable.

The Bacterial Inoculator media (2) has a very important characteristic that differs this new Treatment System in Base on a Bacterial Inoculator (INOCULOM) from the others: it never gets overwhelmed due to the bacterial flora and earthworms in the inoculation medium (sawdust and humus) which are constantly moving and making channels in the medium, which allows it to be always porous and permeable. The solid inorganic materials that come with the served water, which may overwhelm or cover other filters, in this case, are digested by the earthworms and the bacterial flora.

### c) Low operational costs.

This new system of water treatment based on the Bacterial Inoculation (INOCULOM) needs only impulsion pumps to distribute homogeneously the contaminated water over the upper surface of the Bacterial Inoculator (INOCULOM), drastically reducing the energy costs in comparison to the traditional biological treatment systems, as the air or oxygen injection as well as chemicals, which need to be added as well as other substances to the contaminated water.

### d) It produces a subproduct that can be used as natural fertilizer.

Due that earthworms are used in the inoculation medium, these degrade the organic material transforming it in earthworm's humus, which is a natural fertilizer, that is extracted from the Inoculator after some time.

The **DISADVANTAGE** of this new water treatment system based on Bacterial Inoculation (INOCULOM) is the following:

### a) It is sensitive to organic charge or liquid industrial residues variations in the served water.

The Bacterial Inoculation (INOCULOM) biological treatment system, is sensitive to the variations in organic charges, that may be produced at the affluent of this new treatment system (INOCULOM). Any considerable discharge of any chemical toxic substance, as for example: acids, hydrocarbons, inorganic overcharge, etc., may damage this new treatment system (INOCULOM), killing the earthworms and bacterial flora.

The chemical products that are commonly used in homes, for cloth and bathroom cleansing, do not damage this new treatment system (INOCULOM) as a considerable amount is not discharged, because it is diluted in the rest of the used water.

The above describes some preferred embodiments of the present invention but various modifications can be made without departing from the spirit or scope of the invention as defined in the following claims.

The term "comprising" as used in this specification (including the claims, means "consisting at least in part of". Related terms such as "comprise(s)" and "comprised" are to be interpreted in similar manner.

## Claims

1. A system to form different bacterial floras that serves as a decontamination treatment of residual waters, that is **identified** because it uses **EARTHWORM'S HUMUS** of any specie of earthworm, as a detonator of the different bacterial flora that may form depending of the type of residual water that is pretended to decontaminate, which is located in a celluloid support material in the first layer of the system as a support means for the stuck bacterial growth, inert material on the second layer of the system (intermediate) that can be stones or plastic, that may permit the formation of a bacterial flora the supports the degradation of the organic material and finally, on the inferior part (third layer) other inert material that allows to form an air chamber over the system floor, for which it is connected to the surface by vertical tubes through the different layers and horizontal tubes that are connected to the exterior through its walls of the system, this last layer can be made with prefabricated concrete material, stones of different sizes or plastic, that allow an aerobic material and a fast drainage of the water to the exterior of the system.

2. A system to form different bacterial floras that serve as a treatment of residual water decontamination according to the vindication N°. 1 **characterized** because the earthworm humus that is added to the system also includes adult, juvenile, larvae and earthworm's eggs, that allows to keep increasing the humus amount in the system, which feed of the organic solids that are retained by the celluloid material and as of this way they transform the organic material that contains the water to decontaminate the humus, which is an excellent natural fertilizer for lands.

3. A system to form different bacterial flora that serves as a decontamination treatment of residual waters according to vindication N°1 **characterized** because it occupies a previous filtering through a celluloid material, that is to say, sawdust or shaving. When the water that is going to be decontaminated presents a great amount of suspended solids, organics as well as inorganic.

4. A system to form different bacterial floras that serve as a decontamination treatment of residual waters according to the vindication N°1 **characterized** because not all unstable muds are residues.

5. a system to form different bacterial floras that serves as a decontamination treatment of residual waters according to vindication N°1 **characterized** because it contains an irrigation system that permits the contact of the contaminated water with the earthworms' humus so as of this way it can be easier the development of the bacterial flora generated in the system.

6. A system to form different bacterial flora that serves as a decontamination treatment of residual waters according to vindication N°4 **characterized** because it is a natural recycling, that does not accumulate water in order to apply oxygen or chemical products, nor leaves any toxic residue as it is the case of other treatment systems, that leave a contaminating mud as residue of the process of decontamination.

7. An apparatus for treating waste water or other waste liquids (including semi-liquids), the apparatus comprising :
a tank or other container, said container containing:
a first layer comprising humus of earthworms;
a second layer comprising a cellulose or celluloid material, for example, sawdust, underlying the first layer: and
a third layer comprising a substantially inert material defining a porous bed
underlying the second layer;
means to distribute the waste liquid onto the first layer for the waste liquid to perculate through the layers to the bottom of the container; and
means to drain or draw treated liquid from the container.

8. An apparatus as claimed in claim 7, wherein the apparatus includes means to aerate the container, particularly a selected part or parts of the container, more particularly the bottom region of the container.

9. A system to form different bacterial floras that serve as a decontamination treatment of residual waters, that is **characterised** because it uses EARTHWORM'S HUMUS of any specie of earthworm, as a detonator of the different bacterial flora that may form depending of the type of residual water that is pretended to decontaminate, which is located in the first layer of the system, being this last one over a celluloid material which is the second layer that works as a medium for the bacterial growth and a formation of a bacterial flora. On the third layer of the system there's inert material that can be stones or plastic that allow as well the formation of a bacterial flora that helps the degradation of organic material; this three last layers are found inside the tank; the tank's base is made up of inert material that allows taking an air chamber over the system's floor (false bottom) which is connected to the surface by vertical tubes through the different layers and horizontal tubes that are connected to the exterior through the walls of the system; this last layer can be made with prefabricated concrete material, stones of different sizes or plastic, that may allow an aerobic material and a fast drainage of the water to the exterior of the system.

10. An apparatus or system as claimed in any preceding claim, wherein the humus preferably includes earthworms.

11. An apparatus or system as claimed in any preceding claim and substantially as herein described with reference to any embodiment disclosed.

12. An apparatus or system substantially as herein described with reference to any embodiment shown in the accompanying drawings.

13. A method of treating liquid wastes using the apparatus or system as defined in any preceding claim.
